(19) Europäisches Patentamt
European Patent Office
Office européen des brevets

(11) Veröffentlichungsnummer: **0 234 255 B1**

(12) **EUROPÄISCHE PATENTSCHRIFT**

(45) Veröffentlichungstag der Patentschrift: **20.03.91**

(51) Int. Cl.[5]: **C07D 233/16**, C07D 249/08, C07D 403/12, A01N 47/38

(21) Anmeldenummer: **87100676.3**

(22) Anmeldetag: **20.01.87**

# (54) N-(Alkylamino)-azolylharnstoffe und diese enthaltende Fungizide.

(30) Priorität: **23.01.86 DE 3601928**

(43) Veröffentlichungstag der Anmeldung:
**02.09.87 Patentblatt 87/36**

(45) Bekanntmachung des Hinweises auf die Patenterteilung:
**20.03.91 Patentblatt 91/12**

(84) Benannte Vertragsstaaten:
**AT BE CH DE ES FR GB GR IT LI NL SE**

(56) Entgegenhaltungen:
**EP-A- 0 140 194**
**DE-A- 3 411 388**
**GB-A- 2 159 148**

(73) Patentinhaber: **BASF Aktiengesellschaft**
**Carl-Bosch-Strasse 38**
**W-6700 Ludwigshafen(DE)**

(72) Erfinder: **Karbach, Stefan**
**Sperlinggasse 3**
**W-6700 Ludwigshafen(DE)**
Erfinder: **Rentzea, Costin, Dr.**
**Richard-Kuhn-Strasse 1-3**
**W-6900 Heidelberg(DE)**
Erfinder: **Ammermann, Eberhard, Dr.**
**Sachsenstrasse 3**
**W-6700 Ludwigshafen(DE)**
Erfinder: **Pommer, Ernst-Heinrich, Dr.**
**Berliner Platz 7**
**W-6703 Limburgerhof(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die vorliegence Erfindung betrifft neue N-(Alkylamino)-azolylharnstoffe, Verfahren zu ihrer Herstellang und fungizide Mittel, die diese Verbindungen als Wirkstoff enthalten.

Es ist bekannt, N-Propyl-N-(1-(2,4,6-trichlorphenoxy-ethyl-imidazol-1-carboxamid als Fungizid zu verwenden (GB-14 69 772). Seine Wirkung genügt bei niedrigen Aufwandmengen den Anforderungen der Praxis nicht.

Es wurde nun gefunden, daß N-(Alkylamino)-azolylharnstoffe der Formel

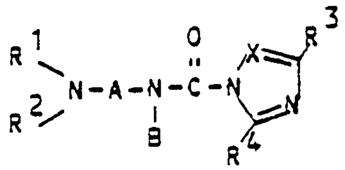

(I),

in der

$R^1$, $R^2$, $R^3$ und $R^4$ gleich oder verschieden sind und Wasserstoff oder Đ$C_1$-$C_6$-Alkyl oder $R^1$ + $R^2$ zusammen mit dem N, dessen Substituenten sie sind, einen gesättigten cyclischen Rest

A eine gegebenenfalls durch $C_1$-$C_4$-Alkyl substituierte $C_1$-$C_6$-Alkylenkette oder einen gegebenenfalls durch $C_1$-$C_4$-Alkyl substituierten Methylen-$C_3$-$C_6$-cycloalkylenring

B gegebenenfalls durch $C_1$-$C_4$-Alkyl substituiertes Arylalkyl, gegebenenfalls durch $C_1$-$C_4$-Alkyl substituiertes Cycloalkyloxyalkyl, gegebenenfalls durch $C_1$-$C_4$-Alkyl substituiertes Cycloalkylmethoxyalkyl, gegebenenfalls durch $C_1$-$C_4$-Alkyl substituiertes Phenoxyalkyl, gegebenenfalls durch $C_1$-$C_4$-Alkyl substituiertes Cyclohexylakyl, $C_4$-$C_6$-Alkylenoxy-$C_7$-$C_{10}$-bycycloalkyl, $C_7$-$C_{10}$-Bicycloalkylmethoxy-$C_4$-$C_6$-alkyl, $C_7$-$C_{10}$-Bicycloalkylenmethoxy-$C_4$-$C_6$-alkyl,

X CH oder N

bedeuten, sehr gut wirksam gegen Schadpilze sind.

Einige neue Verbindungen der Formel I enthalten chirale Zentren und werden im allgemeinen in Form von Mischungen (Racemate oder Diastereomerengemische) erhalten. Die Diastereomeren lassen sich beispielsweise durch Säulenchromatographie trennen oder aufgrund von Löslichkeitsunterschieden in reiner Form isolieren. Aus solchen Diastereomeren oder Racematen kann man mit bekannten Methoden einheitliche Verbindungen erhalten. Diese werden wie ihre Mischungen von der vorliegenden Erfindung umfaßt. Für die Anwendung der neuen Verbindungen als Fungizide sind sowohl die Diastereomeren bzw. Enantiomeren oder die einheitlichen isomeren Verbindungen wie auch deren bei der Synthese anfallenden Gemische geeignet. Bevorzugt werden die letzteren verwendet.

Als Bedeutung für $R^1$, $R^2$, $R^3$ und $R^4$ in Formel I kommen z.B. Wasserstoff, Methyl, Ethyl, n-Propyl, Isopropyl, n-Butyl, Isobutyl, n-Pentyl, Isopentyle, Neopentyl, n-Hexyl, iso-Hexyle in Betracht.

$R^1$ + $R^2$ zusammen bedeuten beispielsweise Methylen oder Polymethylen mit 2 bis 6 C-Atomen, z.B. Tetramethylen. A bedeutet beispielsweise ein gegebenenfalls durch eine oder mehrere Alkylgruppen mit jeweils 1 bis 4 C-Atomen substituiertes $C_1$-$C_6$-Alkylen oder Methylen-$C_3$-$C_6$-cycloalkylen insbesondere Methylencyclobutylen

B bedeutet beispielsweise gegebenenfalls durch $C_1$-$C_4$-Alkyl substituiertes Phenyl-$C_3$-$C_4$-alkyl, $C_1$-$C_4$-Alkylcyclohexyl-$C_1$-$C_4$-alkyl, $C_6$-$C_8$-Cycloalkylmethoxy-$C_4$-$C_6$-alkyl, $C_7$-$C_{10}$-Bicycloalkyl-methoxy-$C_4$-$C_6$-alkyl, $C_7$-$C_{10}$-Bicycloalkenylmethoxy-$C_4$-$C_6$-alkyl, $C_4$-$C_6$-Alkylenoxy substituiert durch $C_5$-$C_{15}$-Cycloalkyl, $C_7$-$C_{10}$-Bicycloalkyl, Phenyl, Phenyl substituiert durch Halogen oder $C_1$-$C_4$-Alkyl

X bedeutet CH oder N.

Die Verbindungen der Formel I lassen sich herstellen, indem man ein Carbamoylchlorid der Formel II

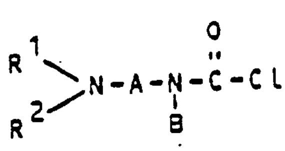

(II),

in der $R^1$, $R^2$, A und B die oben angegebenen Bedeutungen haben,

a) mit Azolen der Formel III

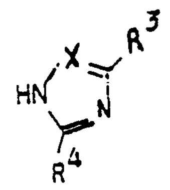

(III),

in der $R^3$, $R^4$ und X die oben angegebenen Bedeutungen haben oder

b) mit deren Metall-Derivaten der Formel IV

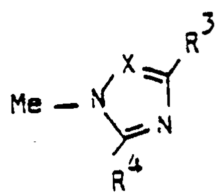

(IV),

in der $R^3$, $R^4$ und X die oben angegebenen Bedeutungen haben und Me Lithium, Natrium, Kalium oder 1/2 Calcium bedeutet, oder

c) mit deren Silicium-Derivaten der Formel V

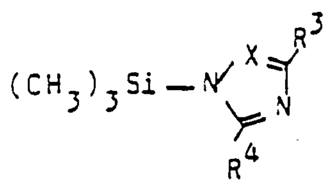

(V),

in der $R^3$, $R^4$ und X die oben angegebenen Bedeutungen haben, umsetzt.

Die Reaktion a) erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120 °C.

Als bevorzugte, gegenüber den Reaktionsteilnehmern, inerte Lösungs-oder Verdünnungsmittel können beispielsweise aliphatische oder aromatische, gegebenenfalls halogenierte Kohlenwasserstoffe, wie n-Pentan, Cyclohexan, Methylenchlorid, 1,1,1-Trichlorethan, Benzol, Toluol, Xylol, Chlorbenzol, aliphatische Ketone, wie Aceton, Methylethylketon, Diethylketon, Ether, wie Diethylether, Methyl-tert.-butylether, Dimethoxyethan, Tetrahydrofuran oder Dioxan, Ester, wie Essigsäureethylester, Nitrile wie Acetonitril, Amide, wie Dimethylformamid, Dimethylacetamid und N-Methylpyrrolidon oder Gemische dieser Lösungsmittel verwendet werden.

Geeignete Basen, die gegebenenfalls auch als säurebindende Mittel bei der Reaktion verwendet werden können, sind beispielsweise Alkalihydroxide wie Lithium-, Natrium-, Kalium- oder Calciumhydroxid, Alkalicarbonate wie Natrium- oder Kaliumcarbonat oder Natrium- und Kaliumhydrogencarbonat, tertiäre Amine wie Triethylamin, Tripropylamin, N-Methylpyrrolidin, N-Methylpiperidin, N,N-Dimethylcyclohexylamin, N,N'-Tetramethylethylendiamin, N,N-Dimethylanilin, N,N-Diethylanilin, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quarternäre Ammoniumsalze wie Tetrabutylammonium-chlorid, -bromid, -iodid und -hydrogensulfat, Benzyltriethylammonium-chlorid oder -bromid oder Kronenether wie 12-Krone-4, 15-Krone-5, 18-Krone-6- oder Dibenzo-18-krone-6-ether in Frage.

Die Reaktionen b) und c) erfolgen gegebenenfalls in Gegenwart eines Lösungs- oder Verdünnungsmittels bei einer Temperatur im Bereich zwischen 0 und 140 °C, vorzugsweise zwischen 0 und 100 °C. Hierfür eignen sich dieselben Lösungsmittel wie für die Verfahrensvariante a).

Die Verbindung der Formel I lassen sich ferner herstellen, indem man eine Verbindung der Formel VI

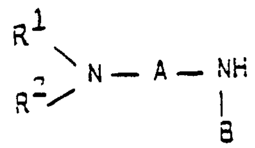

(VI),

in der $R^1$, $R^2$, A und B die oben genannten Bedeutungen haben, mit einem Carbonyl-bisazol der Formel VII

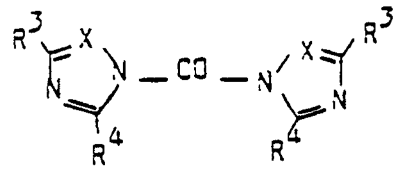

(VII),

in der $R^3$, $R^4$ und X die oben angegebenen Bedeutungen haben, gegebenenfalls in Gegenwart eines Lösungs- oder Verdünnungsmittels, gegebenenfalls unter Zusatz eines Reaktionsbeschleunigers, umsetzt.

Hierfür eignen sich beispielsweise folgende Lösungs- oder Verdünnungsmittel: Diethylether, 1,2-Dimethoxyethan, Dipropylether, Dibutylether, Methyl-tert.-butylether, Tetrahydrofuran, Dimethoxyethan, Anisol, n-Pentan, n-Hexan, n-Heptan, n-Octan, Isooctan, Cyclohexan, Toluol, Chlorbenzol, Xylole, Acetonitril, Essigester, Dimethylformamid, N-Methylpyrrolidon, Aceton oder Methylketon.

Geeignete Reaktionsbeschleuniger sind beispielsweise 4-Dimethylaminopyridin oder 4-Pyrrolidinopyridin.

Die Ausgangsstoffe der Formel II können leicht nach bekannten Verfahren hergestellt werden, beispielsweise durch Umsetzung von Aminen der Formel VI mit Phosgen (Houben-Weyl-Müller, Methoden der organischen Chemie, Band 8, Seiten 115 bis 118, Georg Thieme Verlag, Stuttgart, 1952).

Die folgenden Beispiele erläutern die Herstellung der Verbindungen der Formel I:

Beispiel 1

a) 15 g (0,06 Mol 4-(4-t-butylcyclohexyloxy)-butylchlorid werden mit 30 g (0,34 Mol) Diethylaminoethylamin auf 80 °C erwärmt. Nach 24 Stunden wird abkühlen gelassen, auf 500 ml 2 N HCl gegossen und mit Toluol extrahiert. Die wäßrige Phase wird mit Natronlauge stark alkalisch gestellt und das gebildete Öl wird zweimal mit Ether aus der wäßrigen Phase extrahiert. Nach Trocknen und Einengen der organischen Phase wird das zurückbleibende Öl im Vakuum destilliert. Bei 148 - 152 °C (0,2 mbar) geht 11,8 g gelbliches Öl über, welches IR und NMR spektroskopisch charakterisiert wurde als N-(4-(4-tert.-butylcyclohexyl)-oxybutyl)-N-diethylaminoethylamin.

b) 3,9 g (0,012 Mol) des in a) erhaltenen Amins werden in 50 ml Tetrahydrofuran gelöst und mit 2,9 (0,018 Mol) N,N′-Carbonyldiimidazol versetzt. Nach Rühren über Nacht wurde eingeengt, mit Toluol versetzt und mit $H_2O$ zweimal gewaschen. Nach Trocknen der organischen Phase über Natriumsulfat werden 3,5 g Öl erhalten (Verbindung Nr. 23) mit der Formel

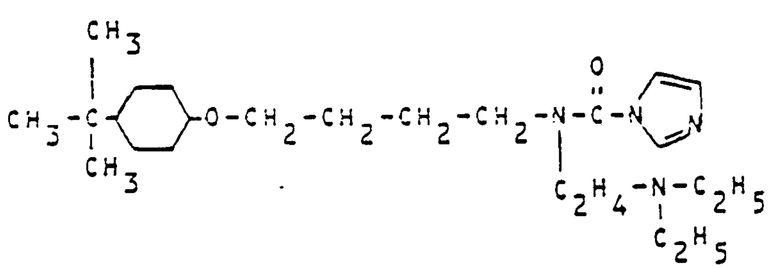

| Nr. | $R^1 + R^2$ | $R^3$ | $R^4$ | A | B | X | $n_D^{22}$ oder IR $cm^{-1}$ |
|---|---|---|---|---|---|---|---|
| 1 | $n-C_4H_9$ | H | H | $C_2H_4$ | 3(4-tert.-Butylphenyl-2--methylprop-1-yl | CH | 3120, 2958, 2931, 2800, 1696, 1378, 1965, 750, 650 |
| 2 | $n-C_4H_9$ | H | H | $C_3H_6$ | " | CH | 3120, 2958, 2933, 2810, 1695, 1798, 365, 750, 650 |
| 3 | $n-C_4H_9$ | H | H | $C_3H_6$ | " | N | 2957, 2931, 2870, 2800, 1700, 1379, 1365, 670 |
| 4 | $n-C_4H_9$ | H | H | $C_2H_4$ | 4-(Bicyclo-[2.2.1]-hept-5-en-methoxy)butyl | | 3050, 2956, 2934, 2800, 1695, 1466, 1421, 1113, 650, 750 |
| 5 | $n-C_4H_9$ | H | H | $C_3H_6$ | " | N | 3060, 2956, 2934, 2800, 1701, 1467, 1114, 670 |
| 6 | $n-C_4H_9$ | H | H | $C_3H_6$ | " | CH | 3060, 2955, 2934, 2800, 1695, 1301, 1274, 750, 650 |
| 7 | $n-C_4H_9$ | H | H | $C_2H_4$ | Tetramethylenoxyhomo-camphanyl | CH | 2356, 2935, 2871, 2800, 1696, 1376, 1365, 750, 635 |
| 8 | $n-C_4H_9$ | H | $i-C_3H_7$ | $C_2H_4$ | " | CH | 2956, 2934, 2871, 2800, 1698, 1376, 1363, 770, 720 |
| 9 | $n-C_4H_9$ | H | H | $C_3H_6$ | " | CH | 2955, 2871, 2799, 1697, 1422, 1365, 750, 650 |

| Nr. | $R^1$ + $R^2$ | $R^3$ | $R^4$ | A | B | X | $n_D^{22}$ oder IR $cm^{-1}$ |
|---|---|---|---|---|---|---|---|
| 10 | n-$C_4H_9$ | H | i-$C_3H_7$ | $C_3H_6$ | " | CH | 2955, 2935, 2800, 1697, 1376, 1364, 1115, 730, 660, 740 |
| 11 | n-$C_4H_9$ | H | H | $C_4H_8$ | " | N | 2954, 2934, 2870, 2800, 1702, 1377, 1364, 1166, 660 |
| 12 | i-$C_3H_7$ | H | H | $C_2H_4$ | 3(4-tert.-Butylphenyl)-2-methylprop-1-yl | CH | 2963, 2931, 2865, 1694, 1463, 1420, 955, 655 |
| 13 | i-$C_3H_7$ | H | H | $C_2H_4$ | " | N | 2964, 2932, 2870, 1699, 1425, 670, 500 |
| 14 | $C_2H_5$ | H | H | $-\overset{CH_3}{CH}-C_2H_4$ | Tetramethylenoxy-pinanyl-10 | CH | 2965, 2935, 2800, 1694, 1414, 1155, 750, 650 |
| 15 | $C_2H_5$ | H | H | $-\overset{CH_3}{CH}-C_2-H_4$ | Tetramethylenoxyhomo-camphanyl | N | 2939, 2870, 2800, 1698, 1422, 1116, 671 |
| 16 | $C_2H_5$ | H | H | $-\overset{CH_3}{CH}-C_2H_4$ | Tetramethylenoxyhomo-camphanyl | CH | 2939, 2870, 2800, 1695, 1414, 1116, 750, 650 |

| Nr. | $R^1$, $R^2$ | $R^3$ | $R^4$ | A | B | X | $n_D^{22}$ oder IR $cm^{-1}$ |
|---|---|---|---|---|---|---|---|
| 17 | $C_2H_5$ | H | H | $-CH_2-C(CH_3)_2-CH_2$ | Tetramethylenoxy-4-n-nonylcyclohexyl | N | 2959, 2933, 2869, 2810, 1703, 1381, 1109, 670 |
| 18 | $C_2H_5$ | H | H | $-CH_2-C(CH_3)_2-CH_2$ | " | CN | - |
| 19 | $C_2H_5$ | H | H | $-CH_2-C(CH_3)_2-CH_2$ | Hexamethylenoxy-4-n-nonylcyclohexyl | CN | - |
| 20 | $C_2H_5$ | H | H | $-C_2H_4-$ | " | CH | 2934, 2860, 2800, 1697, 1422, 1102, 650, 750 |
| 21 | $C_2H_5$ | H | H | $-C_2H_4-$ | " | N | - |
| 22 | $C_2H_5$ | H | H | $-C_2H_4-$ | Tetramethylenoxy-4-t-butylcyclohexyl | N | 2940, 2862, 2810, 1703, 1432, 1276, 920, 670 |
| 23 | $C_2H_5$ | H | H | $-C_2H_4-$ | " | CH | 3110, 2939, 2800, 1698, 1424, 1106, 660 |

| Nr. | $R^1 + R^2$ | $R^3$ | $R^4$ | A | B | X | $n_D^{22}$ oder IR $cm^{-1}$ |
|---|---|---|---|---|---|---|---|
| 24 | $C_2H_5$ | H | H | $-C_2H_4-$ | 3(4-tert.-Butylcyclohexyl)-2-methylprop-1-yl | N | 2935, 2868, 1704, 1434, 1275, 990, 670 |
| 25 | $C_2H_5$ | H | H | $-C_2H_4-$ | " | CH | 2964, 2936, 2800, 1697, 1420, 1239, 760, 660 |
| 26 | $C_2H_5$ | H | H | $C_3H_6$ | 3(4-tert.-Butylphenyl)-2-methylprop-1-yl | CH | 2964, 2934, 2800, 1694, 1420, 1271, 750, 650 |
| 27 | $C_2H_5$ | H | H | $CH_3$<br>$-CH_2-C-CH_2$<br>$CH_3$ | " | N | 2964, 2933, 2810, 1702, 1420, 1275, 670 |
| 28 | $C_2H_5$ | H | H | $CH_3$<br>$-CH_2-C-CH_2-$<br>$CH_3$ | " | CH | 2964, 2871, 2800, 1696, 1414, 1270, 1064, 750, 660 |
| 29 | $C_2H_5$ | H | H | $-C_2H_4-$ | " | CH | 2965, 2835, 2800, 1696, 1419, 1240, 760, 660 |
| 30 | $CH_3$ | H | H | $-C_2H_4-$ | 4-(Cyclohexylmethoxy)-butyl | | 2923, 2852, 2791, 1696, 1449, 1122, 760, 650 |

| Nr. | $R^1 + R^2$ | $R^3$ | $R^4$ | A | B | X | $n_D^{22}$ oder IR $cm^{-1}$ |
|---|---|---|---|---|---|---|---|
| 31 | $CH_3$ | H | H | $-C_3H_6-$ | 4-(Cyclohexylmethoxy)-butyl | | 2925, 2853, 2800, 1695, 1422, 1122, 760, 660 |
| 32 | $CH_3$ | H | H | $-C_4H_8-$ | 4-(Cyclohexylmethoxy)-butyl | | 2924, 2853, 2800, 1694, 1423, 1122, 960, 650 |
| 33 | $CH_3$ | H | H | $-C_3H_6-$ | 3(4-tert.-Butylphenyl-2-methylprop-1-yl | CH | 2960, 2868, 2816, 1694, 1420, 1270, 760, 650 |
| 34 | $CH_3$ | H | H | $-C_4H_8-$ | Tetramethylenoxyhomocamphyl-2- | CH | 2944, 2868, 2810, 1695, 1423, 1282, 1116, 760, 650 |
| 35 | $CH_3$ | H | H | $-C_2H_4-$ | 4-(Bicyclo-[2.2.1]-hept-5-en-methoxy)-butyl | | 2955, 2863, 2800, 1695, 1422 1113, 750, 650 |
| 36 | $CH_3$ | H | H | 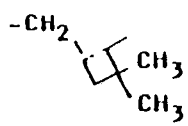 | Tetramethylenoxy-pinanyl-10- | CH | 2946, 2864, 2800, 1695, 1420, 1160, 750, 650 |
| 37 | $CH_3$ | H | H | 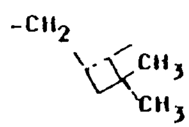 | Tetramethylenoxy-4-t--butylcyclohexyl | N | 2946, 2861, 2768, 1700, 1373, 2366, 670 |
| 38 | $CH_3$ | H | H | 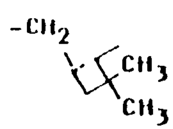 | Tetramethylenoxycyclooctyl | N | 2926, 2860, 2780, 1699, 1276, 1095, 670 |

| Nr. | $R^1$, $R^2$ | $R^3$ | $R^4$ | A | B | X | $n_D^{22}$ oder IR $cm^{-1}$ |
|---|---|---|---|---|---|---|---|
| 39 | $CH_3$ | H | H | $-CH_2$ 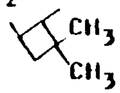 | Tetramethylenoxycyclooctyl | CH | 2926, 2859, 2785, 1695, 1420, 1098, 760, 660 |
| 40 | $CH_3$ | H | H | $-CH_2$ 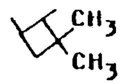 | Tetramethylenoxy-4-t-butyl-cyclohexyl | CH | 2945, 2860, 2785, 1695, 1420, 1107, 760, 660 |
| 41 | $C_2H_5$ | H | H | $-C_2H_4-$ | 4-Phenoxy-butyl | CH | 1,5335 |
| 42 | $C_2H_5$ | H | H | $-C_2H_4-$ | 4-(2-Chlorphenoxy)-butyl | CH | 1,5412 |
| 43 | $C_2H_5$ | H | H | $-C_2H_4-$ | 4-(2-Chlorphenoxy)-butyl | CH | 1,5438 |
| 44 | $C_2H_5$ | H | H | $-C_2H_4-$ | 4-(2-Fluorphenoxy)-butyl | CH | 1,5233 |
| 45 | $C_2H_5$ | H | H | $-C_2H_4-$ | 4-(4-Methylphenoxy)-butyl | CH | 1,5302 |
| 46 | $-CH_2-CH_2-CH_2-CH_2-$ | H | H | $-C_2H_4-$ | 4-Phenoxy-butyl | CH | 1,5452 |
| 47 | $C_2H_5$ | H | H | $-C_2H_4-$ | 3(4-tert.-Butylphenyl)-2--methylprop-1-yl | CH | 1,5212 |
| 48 | $CH_3$ | H | H | $-C_4H_8-$ | 3(4-tert.-Butylphenyl)-2--methylprop-1-yl | CH | 1,5236 |
| 49 | $C_2H_5$ | H | H | $-C_2H_4-$ | 3(2,4-Dichlorphenyl)-2-methylprop-1-yl | CH | Harz |
| 50 | $CH_3$ | H | H | $-C_4H_8-$ | 4-Phenoxy-butyl | CH | |
| 51 | $CH_3$ | $CH_3$ | $CH_3$ | $-C_4H_8-$ | 4-(4-Chlorphenoxy)-butyl | CH | |
| 52 | $CH_3$ | $CH_3$ | $CH_3$ | $-C_4H_8-$ | 4-(4-Chlorphenoxy)-butyl | N | |

| Nr. | $R^1$ + $R^2$ | $R^3$ | $R^4$ | A | B | X | $n_D^{22}$ oder IR $cm^{-1}$ |
|---|---|---|---|---|---|---|---|
| 53 | $CH_3$ | H | H | $-C_4H_8-$ | 4-(4-Chlorphenoxy)-butyl | CH | |
| 54 | $C_2H_5$ | H | H | $-C_4H_8-$ | 4-(2,4,6-Trichlor-phenoxy)-butyl | N | |
| 55 | $C_2H_5$ | H | H | $-C_4H_8-$ | 4-(2,4,6-Trichlor-phenoxy)-butyl | CH | |
| 56 | $C_2H_5$ | H | H | $-C_2H_4-$ | 4-/2,4,6-Trichlor-phenoxy)-butyl | N | |
| 57 | $C_2H_5$ | H | H | $-C_2H_4-$ | 4-(2,4,6-Trichlor-phenoxy)-butyl | CH | |
| 58 | $C_2H_5$ | H | H | $-C_2H_4-$ | 2-(2,4,6-Trichlor-phenoxy)-ethyl | CH | |
| 59 | $C_2H_5$ | H | H | $-C_2H_4-$ | 2-(2,4,6-Trichlor-phenoxy)-ethyl | N | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten, Phycomyceten und Basidiomyceten, aus. Sie sind zum Teil systemisch wirksam und können als Blatt- und Bodenfungizide eingesetzt werden.

Einige der Verbindungen können auch zum Schutz von Materialien zur Bekämfpung holzzerstörender Pilze, wie Coniophora puteana und Polystictus versicolor, eingesetzt werden. Als fungizid wirksame Bestandteile lösemittelhaltiger Holzschutzmittel können sie in der Weise angewendet werden, daß man das Holz beispielsweise mit diesen Mitteln tränkt oder anstreicht. Ferner sind einige der neuen Verbindungen auch gegenüber hauptpathogenen Pilzen, wie Candida albicans und Trichophyton mentagrophytes, wirksam.

Besonders interessant sind die fungiziden Verbindungen für die Bekämpfung einer Vielzahl von Pilzen an verschiedenen Kulturpflanzen oder ihren Saatgütern insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis an Getreide,
Podosphaera leucotricha an Äpfeln,
Uncinula necator an Reben,
Puccinia-Arten an Getreide,
Ustilago-Arten an Getreide und Zuckerrohr,
Venturia inaequalis (Schorf) an Äpfeln,
Septoria nodorum an Weizen,
Pyrenophora teres an Gerste
Botrytis cinerea (Grauschimmel) an Erdbeeren, Reben,
Pseudocercosporella herpotrichoides an Weizen, Gerste,
Pyricularia oryzae an Reis,
Alternaria solani an Kartoffeln, Tomaten
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen oder die Fläche (Bodenfläche) mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 3 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes und 40 Mol Ethylenoxid an 1 Mol Ricinusöl besteht. Durch Ausgießen und feines

Verteilen der Lösung in Wasser erhält man eine wäßrige Dispersion.

III. 20 Gewichtsteile der Verbindung Nr. 8 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion, die 0,02 Gew.% des Wirkstoffs enthält.

IV. 20 Gewichtsteile der Verbindung Nr. 9 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 13 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 15 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 25 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 26 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 27 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Für den folgenden Versuch wurde als Vergleich der bekannte Wirkstoff N-Propyl-N-(2.4.5-Trichlorphenoxy-ethyl)-imidazol-1-carboxamid (A) verwendet.

Anwendungsbeispiel

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 8 Tage im Gewächshaus aufgestellt. Erst dann werden die Blätter mit einer Zoosporenaufschwemmung von Plasmopara viticola (Rebenperonospora) infiziert. Danach werden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß die Wirkstoffe Nr. 2, 3, 8, 9, 13, 15, 25, 26, 27, 37 und 58 bei der Anwendung als 0,05%ige (Gew.%) Spritzbrühe eine bessere fungizide Wirkung (97 %) zeigen als der bekannte Wirkstoff A (70 %).

## Ansprüche

**1.** N-(Alkylamino)-azolyharnstoffe der Formel

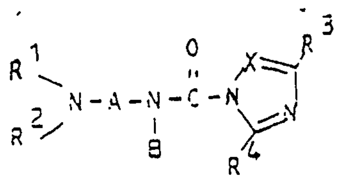

(I),

in der
$R^1$, $R^2$, $R^3$ und $R^4$ gleich oder verschieden sind und Wasserstoff oder $R^1$ und $R^2$ zusammen mit dem N, dessen Substituenten sie sind, einen gesättigten cyclischen Rest
A eine gegebenenfalls durch $C_1$-$C_4$-Alkyl substituierte $C_1$-$C_4$-Alkylenkette oder einen gegebenenfalls durch $C_1$-$C_4$-Alkyl substituierten Methylen-$C_3$-$C_6$-cycloalkylenring
B gegebenenfalls durch $c_1$-$C_4$-Alkyl substituiertes Arylalkyl, gegebenenfalls durch $C_1$-$C_4$-Alkyl substituiertes Cycloalkyloxyalkyl, gegebenenfalls durch $C_1$-$C_4$-Alkyl substituiertes Cycloalkylmethoxyalkyl, gegebenenfalls durch $C_1$-$C_4$-Alkyl substituiertes Phenoxyalkyl gegebenenfalls durch $C_1$-$C_4$-Alkyl substituiertes Cyclohexylalkyl, $C_4$-$C_6$-Alkylenoxy-$C_7$-$C_{10}$-bicycloalkyl, $C_7$-$C_{10}$-Bicycloalkylmethoxy-$C_4$-$C_6$-alkyl, $C_7$-$C_{10}$-Bicycloalkylenmethoxy-$C_4$-$C_6$-alkyl,
X CH oder N
bedeuten.

**2.** N-(Alkylamino)-azolylharnstoffe der Formel 1 gemäß Anspruch 1 dadurch gekennzeichnet, daß
$R^1$, $R^2$, $R^3$ und $R^4$ Wasserstoff, Methyl, Ethyl, n-Propyl, Isopropyl, n-Butyl, 2-Butyl, n-Pentyl, iso-Pentyle, Neopentyl-, n-Hexyl sowie iso-Hexyle bedeuten und gleich oder verschieden sind.
A ein gegebenenfalls durch eine oder mehrere Alkylgruppen mit jeweils 1 bis 3 C-Atomen substituiertes $C_1$-$C_6$-Alkylen oder Cyclobutylen
B gegebenenfalls durch $C_1$-$C_4$-Alkyl substituiertes Phenyl-$C_3$-$C_4$-alkyl, $C_1$-$C_4$-Alkylcyclohexyl-$C_1$-$C_4$-alkyl, $C_6$-$C_8$-Cycloalkylmethoxy-$C_4$-$C_6$-alkyl, $C_7$-$C_{10}$-Bicycloalkyl-methoxy-$C_4$-$C_6$-alkyl, $C_7$-$C_{10}$-Bicycloalkenylmethoxy-$C_4$-$C_6$-alkyl, $C_4$-$C_6$-Alkylenoxy substituiert durch $C_6$-$C_{15}$-Cycloalkyl, $C_7$-$C_{10}$-Bicycloalkyl, Phenyl, Phenyl substituiert durch Halogen oder $C_1$-$C_4$-Alkyl
X CH oder N bedeuten.

**3.** Fungizides Mittel, enthaltend einen festen oder flüssigen Trägerstoff und einen N-(Alkylamino)-azolylharnstoff der Formel 1

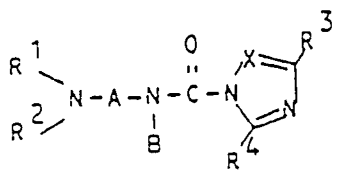

(I),

in der
$R^1$, $R^2$, $R^3$ und $R^4$ gleich oder verschieden sind und Wasserstoff oder $C_1$-$C_4$-Alkyl oder $R^1$ und $R^2$ zusammen mit dem N, dessen Substituenten sie sind, einen gesättigten cyclischen Rest
A eine gegebenenfalls durch $C_1$-$C_4$-Alkyl subituierte $C_1$-$C_4$-Alkylenkette oder einen gegebenenfalls durch $C_1$-$C_4$-Alkyl substituierten Methylen-$C_3$-$C_6$-Cycloalkylenring
B gegebenenfalls durch $C_1$-$C_4$-Alkyl substituiertes Arylalkyl, gegebenenfalls durch $C_1$-$C_4$-Alkyl substituiertes Cycloalkyloxyalkyl, gegebenenfalls durch $C_1$-$C_4$-Alkyl substituiertes Cycloalkylmethoxyalkyl, gegebenenfalls durch $C_1$-$C_4$-Alkyl substituiertes Phenoxyalkyl, gegebenenfalls durch $C_1$-$C_4$-Alkyl substituiertes Cyclohexylalkyl, $C_4$-$C_6$-Alkylenoxy-$C_7$-$C_{10}$-bicycloalkyl, $C_7$-$C_{10}$-Bicycloalkylmethoxy-$C_4$-$C_6$-alkyl, $C_7$-$C_{10}$-Bicycloalkylenmethoxy-$C_4$-$C_6$-alkyl, X CH oder N
bedeuten.

**4.** Verfahren zur Bekämpfung von Pilzen, dadurch gekennzeichnet, daß man einen N-(Alkylamino)-azolylharnstoff der Formel I

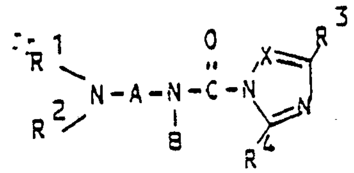

(I),

in der

$R^1$, $R^2$, $R^3$ und $R^4$ gleich oder verschieden sind und Wasserstoff oder $C_1$-$C_4$-Alkyl oder $R^1$ und $R^2$ zusammen mit dem N, dessen Substituenten sie sind, einen gesättigten cyclischen Rest

A eine gegebenenfalls durch $C_1$-$C_4$-Alkyl substituierte $C_1$-$C_6$-Alkylenkette oder einen gegebenenfalls durci $C_1$-$C_4$-Alkyl substituierten Methylen-$C_3$-$C_6$-cycloalkylenring

B gegebenenfalls durch $C_1$-$C_4$-Alkyl substituiertes Arylalkyl, gegebenenfalls durch $C_1$-$C_4$-Alkyl substituiertes Cycloalkyloxyalkyl, gegebenenfalls durch $C_1$-$C_4$-Alkyl substituiertes Cycloalkylmethoxyalkyl, gegebenenfalls durch $C_1$-$C_4$-Alkyl substituiertes Phenoxyalkyl, gegebenenfalls durch $C_1$-$C_4$-Alkyl substituiertes Cyclohexylalkyl, $C_4$-$C_6$-Alkylenoxy-$C_7$-$C_{10}$-bicycloalkyl, $C_7$-$C_{10}$-Bicycloalkylmethoxy-$C_4$-$C_6$-alkyl, $C_7$-$C_{10}$-Bicycloalkylenmethoxy-$C_4$-$C_6$-alkyl,

X CH oder N

bedeuten,

auf die Pilze oder auf durch Pilzbefall bedrohte Materialien, Flächen, Pflanzen oder Saatgüter einwirken läßt.

5. Verfahren zur Herstellung eines fungiziden Mittels, dadurch gekennzeichnet, daß man einen N-(Alkylamino)-azolyharnstoff der Formel I gemäß Anspruch 1 mit einem festen oder flüssigen Trägerstoff mischt.

6. Verbindung der Formel I gemäß Anspruch 1, in der $R^1$ und $R^2$ Butyl, $R^3$ und $R^4$ Wasserstoff, A Propyl und B 3-(4-tert.-Butylphenyil)-2-methyl-prop-1-yl und X N bedeutet.

7. Verbindung der Formel I gemäß Anspruch 1, in der $R^1$ und $R^2$ Butyl, $R^3$ und $R^4$ Wasserstoff, A Propyl, und B 3-(4-tert.-Butylphenyl)-2-methyl-prop-1-yl und X CH bedeutet.

8. Verbindung der Formel I gemäß Anspruch 1, in der $R^1$ und $R^2$ Butyl, $R^3$ Wasserstoff, $R^4$ Isopropyl, A Ethyl, B Tetramethylenoxyhomocamphanyl und X CH bedeutet.

9. Verbindung der Formel I gemäß Anspruch 1, in der $R^1$ und $R^2$ Butyl, $R^3$ und $R^4$ Wasserstoff, A Propyl, B Tetramethylenoxyhomocamphanyl und X CH bedeutet.

10. Verbindung der Formel I gemäß Anspruch 1, in der $R^1$ und $R^2$ Isopropyl, $R^3$ und $R^4$ Wasserstoff, A Ethyl, B 3-(4-tert.-Butylphenyl)-2-methyl-prop-1-yl und X N bedeutet.

## Claims

1. An N-(alkylamino)-azolylurea of the formula

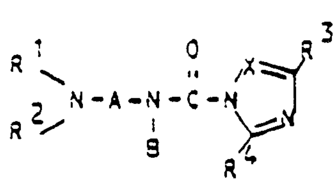

(I)

where $R^1$, $R^2$, $R^3$ and $R^4$ are identical or different and are each hydrogen or $C_1$-$C_4$-alkyl or $R^1$ and $R^2$, together with the nitrogen atom whose substituents they are, form a saturated cyclic radical,

A is an unsubstituted or $C_1$-$C_4$-alkyl-substituted $C_1$-$C_6$-alkylene chain or an unsubstituted or $C_1$-$C_4$-alkyl-sub-stituted methylene-$C_3$- or $C_4$-cycloalkylene ring,

B is unsubstituted or $C_1$-$C_4$-alkyl-substituted aryl-alkyl, unsubstituted or $C_1$-$C_4$-alkyl-substituted cycloal-

kyloxyalkyl, unsubstituted or $C_1$-$C_4$-alkyl-substituted cycloalkylmethoxyalkyl, unsubstituted or $C_1$-$C_4$-alkyl-substituted phenoxyalkyl, unsubstituted or $C_1$-$C_4$-alkyl-substituted cyclohexylalkyl, $C_4$-$C_6$-alkylenoxy-$C_7$-$C_{10}$-bicycloalkyl, $C_7$-$C_{10}$-bicycloalkylmethoxy-$C_4$-$C_6$-alkyl or $C_7$-$C_{10}$-bicycloalkylenemethoxy-$C_4$-$C_6$-alkyl, and
X is CH or N.

2. An N-(alkylamino)-azolylurea of the formula I as claimed in claim 1, wherein $R^1$, $R^2$, $R^3$ and $R^4$ are each hydrogen, methyl, ethyl, n-propyl, isopropyl, n-butyl, 2-butyl, n-pentyl, one of the isopentyl radicals, neopentyl, n-hexyl or one of the isohexyl radicals and are identical or different,
A is cyclobutylene or $C_1$-$C_6$-alkylene which is unsubstituted or substituted by one or more alkyl groups, each of 1 to 3 carbon atoms, and
B is unsubstituted or $C_1$-$C_4$-alkyl-substituted phenyl$C_3$- or -$C_4$-alkyl, $C_1$-$C_4$-alkylcyclohexyl-$C_1$-$C_4$-alkyl, $C_6$-$C_8$-cycloalkylmethoxy-$C_4$-$C_6$-alkyl, $C_7$-$C_{10}$-bicycloalkylmethoxy-$C_4$-$C_6$-alkyl, $C_7$-$C_{10}$-bicycloalkenylmethoxy-$C_4$-$C_6$-alxyl, $C_4$-$C_6$-alkylenoxy substituted by $C_6$-$C_{15}$-cycloalkyl, $C_7$-$C_{10}$-bicycloalkyl, phenyl, phenyl substituted by halogen or $C_1$-$C_4$-alkyl, and
X is CH or N.

3. A fungicide containing a solid or liquid carrier and an N-(alkylamino)-azolylurea of the formula I

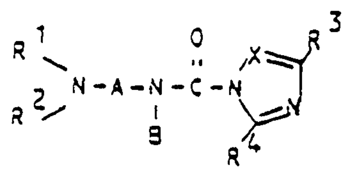
(I),

where $R^1$, $R^2$, $R^3$ and $R^4$ are identical or different and are each hydrogen or $C_1$-$C_4$-alkyl or $R^1$ and $R^2$, together with the nitrogen atom whose substituents they are, form a saturated cyclic radical,
A is an unsubstituted or $C_1$-$C_4$-alkyl-substituted $C_1$-$C_6$-alkylene chain or an unsubstituted $C_1$-$C_4$-alkyl-substituted methylene-$C_3$- or $C_4$-cycloalkylene ring,
B is unsubstituted or $C_1$-$C_4$-alkyl-substituted arylalkyl, unsubstituted or $C_1$-$C_4$-alkyl-substituted cycloalkyloxyalkyl, unsubstituted or $C_1$-$C_4$-alkyl-substituted cycloalkylmethoxyalkyl, unsubstituted or $C_1$-$C_4$-alkyl-substituted phenoxyalkyl, unsubstituted or $C_1$-$C_4$-alkyl-substituted cyclohexylalkyl, $C_4$-$C_6$-alkylenoxy-$C_7$-$C_{10}$-bicycloalkyl, $C_7$-$C_{10}$-bicycloalkylmethoxy-$C_4$-$C_6$-alxyl or $C_7$-$C_{10}$-bicycloalkylenemethoxy-$C_4$-$C_6$-alkyl, and
X is CH or N.

4. A method for controlling fungi, wherein an N-(alkylamino)-azolylurea of the formula I

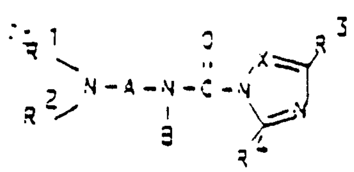
(I),

where $R^1$, $R^2$, $R^3$ and $R^4$ are identical or different and are each hydrogen or $C_1$-$C_4$-alkyl or $R^1$ and $R^2$, together with the nitrogen atom whose substituents they are, form a saturated cyclic radical,
A is an unsubstituted or $C_1$-$C_4$-alkyl-substituted $C_1$-$C_6$-alkylene chain or an unsubstituted or $C_1$-$C_4$-alkyl-substituted methylene-$C_3$- or $C_4$-cycloalkylene ring,
B is unsubstituted or $C_1$-$C_4$-alkyl-substituted arylalkyl, unsubstituted or $C_1$-$C_4$-alkyl-substituted cycloalkyloxyalkyl, unsubstituted or $C_1$-$C_4$-alkyl-substituted cycloalkylmethoxyalkyl, unsubstituted or $C_1$-$C_4$-alkyl-substituted phenoxyalkyl, unsubstituted or $C_1$-$C_4$-alkyl-substituted cyclohexylalkyl, $C_4$-$C_6$-alkylenoxy-$C_7$-$C_{10}$-bicycloalkyl, $C_7$-$C_{10}$-bicycloalkylmethoxy-$C_4$-$C_6$-alkyl or $C_7$-$C_{10}$-bicycloalkylenemethoxy-$C_4$-$C_6$-alkyl, and
X is CH or N,

is allowed to act on the fungi or on materials, surfaces, plants or seeds threatened by fungal attack.

5. A process for the preparation of a fungicide, wherein an N-(alkylamino)-azolylurea of the formula I as claimed in claim 1 is mixed with a solid or liquid carrier.

6. A compound of the formula I as claimed in claim 1, where $R^1$ and $R^2$ are each butyl, $R^3$ and $R^4$ are each hydrogen, A is propyl, B is 3-(4-tert-butylphenyl)-2-methylprop-1-yl and X is N.

7. A compound of the formula I as claimed in claim 1, where $R^1$ and $R^2$ are each butyl, $R^3$ and $R^4$ are each hydrogen, A is butyl, B is 3-(4-tert-butylphenyl)-2-methylprop-1-yl and X is CH.

8. A compound of the formula I as claimed in claim 1, where $R^1$ and $R^2$ are each butyl, $R^3$ is hydrogen, $R^4$ is isopropyl, A is ethyl, B is tetramethyleneoxyhomocamphanyl and X is CH.

9. A compound of the formula I as claimed in claim 1, where $R^1$ and $R^2$ are each butyl, $R^3$ and $R^4$ are each hydrogen, A is propyl, B is tetramethyleneoxyhomocamphanyl and X is CH.

10. A compound of the formula I as claimed in claim 1, where $R^1$ and $R^2$ are each isopropyl, $R^3$ and $R^4$ are each hydrogen, A is ethyl, B is 3-(4-tert-butylphenyl)-2-methylprop-1-yl and X is N.

## Revendications

1. N-(alkylamino)-azolylurées de formule

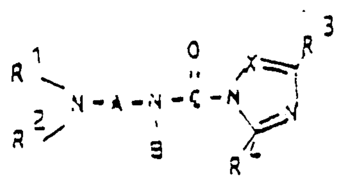

(I),

dans laquelle
$R^1$, $R^2$, $R^3$ et $R^4$, ayant des significations identiques ou différentes, représentent chacun l'hydrogène ou un groupe alkyle en C 1-C 4, ou bien $R^1$ et $R^2$ représentent ensemble et avec l'atome d'azote auquel ils sont reliés un groupe cyclique saturé,
A représente une chaîne alkylène en C 1-C 4 éventuellement substituée par un groupe alkyle en C 1-C 4 ou un noyau méthylène-cycloalkylene en C 1-C 6 éventuellement substitué par un groupe alkyle en C 1- C 4,
B représente un groupe arylalkyle éventuellement substitué par un groupe alkyle en C 1-C 4, un groupe cycloalkyloxyalkyle éventuellement substitué par un groupe alkyle en C 1-C 4, un groupe cycloalkylméthoxyalkyle éventuellement substitué par un groupe alkyle en C 1-C 4, un groupe phénoxyalkyle éventuellement substitué par un groupe alkyle en C 1-C 4, un groupe cyclohexylalkyle, (alkylène en C 4-C 6)-oxybicycloalkyle en C 7-C 10, (bicycloalkyle en C 7-C 10)-méthoxy-alkyle en C 4-C 6, (bicycloalkylène en C 7-C 10)-méthoxy-alkyle en C 4-C 6, éventuellement substitué par un groupe alkyle en C 1-C 4,
X représente CH ou N.

2. N-(alkylamino)-azolylurées de formule I de la revendication 1, caractérisées en ce que :
$R^1$, $R^2$, $R^3$ et $R^4$ représentent l'hydrogène, des groupes méthyle, éthyle, n-propyle, isopropyle, n-butyle, 2-butyle, n-pentyle, isopentyle, néopentyle, n-hexyle ou isohexyle et ont des significations identiques ou différentes,
A représente un groupe alkylène en C 1-C 6 ou cyclobutylène éventuellement substitué par un ou plusieurs groupes alkyle contenant chacun 1 à 3 atomes de carbone,
B représente un groupe phényl-alkyle en C 1-C 4, (alkyle en C 1-C 4)-cyclohexylalkyle en C 1-C 4, (cycloalkyle en C 6-C 8)-méthoxy-alkyle en C 4-C 6, (bicycloalkyle en C 7-C 10)-méthoxy-alkyle en C 4-C 6, (bicycloalcényle en C 7-C 10)-méthoxy-alkyle en C 4-C 6 éventuellement substitué par un groupe alkyle en C 1-C 4, un groupe alkylène-oxy en C 1-C 6 substitué par un groupe cycloalkyle en C

6-C 15, un groupe bicycloalkyle en C 7-C 10, phényle, phényle substitué par des halogènes ou des groupes alkyle en C 1-C 4,
X représente CH ou N.

3. Produit fongicide contenant un véhicule solide ou liquide et une N-(alkylamino)-azolylurée de formule I

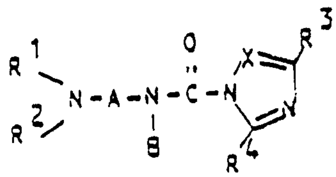

(I),

dans laquelle
$R^1$, $R^2$, $R^3$ et $R^4$, ayant des significations identiques ou différentes, représentent chacun l'hydrogène ou un groupe alkyle en C 1-C 4, ou bien $R^1$ et $R^2$ forment ensemble et avec l'atome d'azote auquel ils sont reliés un noyau cyclique saturé,
A représente une chaîne alkylène en C 1-C 6 éventuellement substituée par un groupe alkyle en C 1-C 4 ou un noyau méthylene-cycloalkylène en C 1-C 6 éventuellement substitué par un groupe alkyle en C 1-C 4,
B représente un groupe arylalkyle éventuellement substitué par un groupe alkyle en C 1-C 4, un groupe cycloalkyloxyalkyle éventuellement substitué par un groupe alkyle en C 1-C 4, un groupe cycloalkylméthoxyalkyle éventuellement substitué par un groupe alkyle en C 1-C 4, un groupe phénoxyalkyle éventuellement substitué par un groupe alkyle en C 1-C 4, un groupe cyclohexylalkyle, (alkylène en C 4-C 6)-oxy-bicycloalkyle en C 7-C 10, (bicycloalkyle en C 7-C 10)-méthoxy-alkyle en C 4-C 6, (bicycloalkylène en C 7-C 10)-méthoxy-alkyle en C 4-C 6 éventuellement substitué par un groupe alkyle en C 1-C 4,
X représente CH ou N;

4. Procédé pour combattre les mycètes, caractérisé en ce que l'on fait agir sur les mycètes ou les matériaux, surfaces, plantes ou semences menacés d'une attaque par les mycètes, une N-(alkylamino)-azolylurée de formule I

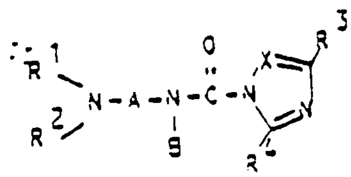

(I),

dans laquelle
$R^1$, $R^2$, $R^3$ et $R^4$, ayant des significations identiques ou différentes, représentent chacun l'hydrogène ou un groupe alkyle en C 1-C 4 ou bien $R^1$ et $R^2$ forment ensemble et avec l'atome d'azote auquel ils sont reliés un noyau cyclique saturé,
A représente une chaîne alkylène en C 1-C 6 éventuellement substituée par un groupe alkyle en C 1-C 4 ou un noyau méthylène-cycloalkylène en C 1-C 6 éventuellement substitué par un groupe alkyle en C 1-C 4,
B représente un groupe arylalkyle éventuellement substitué par un groupe alkyle en C 1-C 4, un groupe cycloalkyloxyalkyle éventuellement substitué par un groupe alkyle en C 1-C 4, un groupe cycloalkylméthoxyalkyle éventuellement substitué par un groupe alkyle en C 1-C 4, un groupe phénoxyalkyle éventuellement substitué par un groupe alkyle en C 1-C 4, un groupe cyclohexylalkyle, (alkylène en C 4-C 6)-oxy-bicycloalkyle en C 7-C 10, (bicycloalkyle en C 7-C 10)-méthoxy-alkyle en C 4-C 6, (bicycloalkylène en C 7-C 10)-méthoxy-alkyle en C 4-C 6 éventuellement substitué par un groupe alkyle en C 1-C 4,
X représente CH ou N.

5. Procédé de préparation d'un produit fongicide, caractérisé en ce que l'on mélange une N-(alkylamino)-azolylurée de formule I de la revendication 1 avec un véhicule solide ou liquide.

6. Composé de formule I de la revendication 1, dans laquelle $R^1$ et $R^2$ représentent chacun un groupe butyle, $R^3$ et $R^4$ représentent chacun l'hydrogène, A représente un groupe propyle et B un groupe 3-(4-tert-butylphényl)-2-méthylprop-1-yle et X représente N.

7. Composé de formule I de la revendication 1, dans laquelle $R^1$ et $R^2$ représentent chacun un groupe butyle, $R^3$ et $R^4$ représentent chacun l'hydrogène, A un groupe propyle et B un groupe 3-(4-tert-butylphényl)-2-méthylprop-1-yle et X représente CH.

8. Composé de formule I selon la revendication 1, dans laquelle $R^1$ et $R^2$ représentent chacun un groupe butyle, $R^3$ l'hydrogène, $R^4$ un groupe isopropyle, A un groupe éthyle, B un groupe tétraméthylène-oxy-homocamphanyle et X représente CH.

9. Composé de formule I selon la revendication 1, dans laquelle $R^1$ et $R^2$ représentent chacun un groupe butyle, $R^3$ et $R^4$ l'hydrogène, A un groupe propyle, B un groupe tétraméthylène-oxy-homocamphanyle et X représente CH.

10. Composé de formule I selon la revendication 1, dans laquelle $R^1$ et $R^2$ représentent chacun un groupe isopropyle, $R^3$ et $R^4$ l'hydrogène, A un groupe éthyle, B un groupe 3-(4-tert-butylphényl)-2-méthylprop-1-yle et X représente N.